# EUROPÄISCHE PATENTSCHRIFT

(11) **EP 1 789 475 B1**
(45) Veröffentlichungstag und Bekanntmachung des Hinweises auf die Patenterteilung: **14.07.2010**
(21) Anmeldenummer: 05786853.1
(22) Anmeldetag: 02.09.2005
(51) Int. Cl.: C08G 79/04, C07D 251/00

(54) **POLYPHOSPHATDERIVAT EINER 1,3,5-TRIAZINVERBINDUNG, VERFAHREN ZU DESSEN HERSTELLUNG UND DESSEN VERWENDUNG**
POLYPHOSPHATE DERIVATIVE OF A 1,3,5-TRIAZINE COMPOUND, METHOD FOR PRODUCING THE SAME AND ITS USE
DERIVE DE POLYPHOSPHATE A PARTIR D'UN COMPOSE DE TRIAZINE 1,3,5, PROCEDE DE PRODUCTION ET D'UTILISATION ASSOCIE

(30) Priorität: 04.09.2004 DE 102004042833
(43) Veröffentlichungstag der Anmeldung: 30.05.2007
(73) Patentinhaber: Chemische Fabrik Budenheim KG, 55257 Budenheim (DE)
(72) Erfinder: NÄGERL, Hans-Dieter, 67373 Dudenhofen (DE); FUTTERER, Thomas, 55218 Ingelheim (DE); MANS FIBLA, Vincens, E-08915 Badalona (ES); GARCIA MARTINEZ, David, E-50013 Zaragoza (ES); TORTOSA GIMENO, Eduardo, E-08029 Barcelona (ES)
(74) Vertreter: WSL Patentanwälte
(86) Internationale Anmeldenummer: PCT/EP2005/054340
(87) Internationale Veröffentlichungsnummer: WO 2006/027340

(56) Entgegenhaltungen:
- EP-A- 0 974 588
- WO-A-97/44377
- WO-A-03/031417

## Beschreibung

Aus der WO 00/02869 sind Polyphosphatsalze von 1,3,5-Triazinverbindungen bekannt, die einen mittleren Kondensationsgrad (Zahlenmittel) größer als 20 und ein Molverhältnis von Triazinverbindung, wie von Melamin, zu Phosphor (M/P) > 1,1 besitzen. Die WO-Schrift beschreibt auch ein zweistufiges Verfahren zur Herstellung dieser Salze durch Umwandlung einer 1,3,5-Triazinvebindung mit Orthophosphorsäure in das entsprechende Orthophosphatsalz sowie thermische Behandlung zur Umwandlung des Orthophosphatsalzes in ein Polyphosphat der 1,3,5-Triazinverbindung. Zusätzlich zu den Orthophosphaten können auch Pyrophosphate eingesetzt werden. Die in dieser Veröffentlichung beschriebenen Polyphosphatsalze sollen zweckmäßig als Flammschutzmittel verwendet werden.

Die WO-Schrift WO 97/44377 beschreibt ein Melaminpolymetaphosphat mit einer Löslichkeit von 0,01 bis 0,10 g/100 ml in Wasser bei 25°C, einem pH-Wert von 2,5 bis 4,5 in der Form einer 10-Gew.-%igen wäßrigen Aufschlämmung bei 25°C und einem Melamingehalt von 1,0 bis 1,1 Mol je Mol Phosphor. Auch dieses Melaminpolymetaphosphat wird in einem zweistufigen Verfahren gewonnen, bei dem man in einer ersten Stufe Melamin, Harnstoff und eine wäßrige Orthophosphorsäurelösung in einem solchen Verhältnis miteinander vermischt, daß das Molverhältnis von Melamin zu Orthophosphorsäure 1,0 bis 1,5 beträgt und das Molverhältnis von Harnstoff zu Orthophosphorsäure 0,1 bis 1,5 beträgt. Die Umsetzung erfolgt bei einer Temperatur von 0 bis 140°C unter Entfernung von Wasser, wobei man ein pulveriges Doppelsalz von Orthophosphorsäure, Melamin und Harnstoff bekommt. In einer zweiten Stufe wird dieses bei einer Temperatur von 240 bis 340°C kalziniert und ergibt dabei Melaminpolymetaphosphate. Auch diese Veröffentlichung nennt als Verwendungsgebiet für die Melaminpolymetaphosphate Flammschutzmittel.

Die WO 00/02869 nennt als Nachteil der Melaminpolymetaphosphate gemäß der WO 97/44377, daß die nach dem dort beschriebenen Verfahren hergestellten Melaminpolymetaphosphate ungeeignet für die Verwendung als Flammschutzmittel in Polymeren, insbesondere in Polyamiden und Polyestern seien, die typischerweise bei erhöhten Temperaturen verarbeitet werden. Diese Melaminpolymetaphosphate haben nach den Angaben in der WO 00/02869 unzureichende Wärmebeständigkeit, Schlagfestigkeit, Zugfestigkeit und Bruchfestigkeit. Es bestand somit für den Fachmann keine Veranlassung, etwa zur Verbesserung der Polyphosphatsalze gemäß der WO 00/02 869 Anregungen der WO 97/44377 zu entnehmen.

Die EP 0 974 588 beschreibt 1,3,5-Triazinderivate von Polysäuren, die Phosphor. Schwefel und Sauerstoff enthalten, und ein Verfahren zu deren Herstellung. Das Verhältnis von 1,3,5-Triazinverbindung zu Phosphor in den offenbarten Triyzinpolyphosphatderivaten ist größer als 1,1. Es werden keine Angaben zu dem mittleren Kondensationsgrad gemacht.

In der WO 03/031417 werden Triazinammoniumcopolyphosphate beschrieben, die durch unterschiedliche Verfahren erhalten werden. Die beschriebenen Copolyphosphate werden als Flammschutzmittel eingesetzt und weisen eine relativ hohe Löslichkeit in Wasser auf.

Aus der EP-OS 1 386 942 sind Flammschutzmittel bekannt, die ein Phosphinat oder Diphosphinat zusammen mit einer 1,3,5-Triazinverbindung enthalten, die die Wirkung gegenüber den Einzelsubstanzen verbessern sollen.

Bei der Verwendung der Polyphosphatsalze gemäß der WO 00/02869 als Flammschutzmittel in Kunststoffen, insbesondere in glasfaserverstärkten Polyamiden, Polyestern, wie Polyethylenterephthalat und Polybutylenterephthalat, die üblicherweise bei relativ hohen Temperaturen (nämlich oberhalb 320°C), verarbeitet werden, stellte man ebenfalls vorzeitige Teilzersetzung fest, die einerseits zu dem Angriff der Zersetzungsprodukte auf die verwendeten Formwerkzeuge und später bei Verwendung beispielsweise als Elektroisolierlacke auf Kupferdrähten zu einem Angriff auf letztere führen.

Die der Erfindung zugrundeliegende Aufgabe bestand somit darin, die aus der WO 00/02869 bekannten Polyphosphatsalze von 1,3.5-Triazinverbindungen weiter zu verbessern, so daß sie auch bei Verarbeitungstemperaturen oberhalb 320°C hitzebeständig sind und dazu geringe Wasserlöslichkeit und geringe Leitfähigkeit besitzen. Überraschenderweise wird diese Aufgabe gelöst durch ein Polyphosphatderivat einer 1,3.5-Triazinverbindung, vorzugsweise Melaminpolyphosphat, mit
a) einem mittleren Kondensationsgrad n (Zahlenmittel) > 20
b) mit einem pH-Wert einer 10%igen Aufschlämmung des Polyphosphatderivates in Wasser bei 25°C von 5 oder höher,
c) einem Molverhältnis von 1,3,5-Triazinverbindung zu Phosphor (M/P) < 1,1,
d) einer Zersetzungstemperatur > 320°C. bei welcher der Gewichtsverlust 2% beträgt, und
e) einer Löslichkeit < 0,1 g/100 ml.

In einer Ausführungsform der Erfindung hat das Polyphosphatderivat eine Löslichkeit < 0,01 g/100 ml.

Diese Polyphosphatderivate können als Flammschutzmittel in beliebigen Kunststoffen, vorzugsweise Thermoplasten und Duroplasten, verwendet werden, insbesondere auch in glasfaserverstärkten Polyamiden und Polyestern, die bei hohen Temperaturen verarbeitet werden. Aufgrund der Angaben in der WO 00/02869 auf den Seiten 1 und 2 sowie 11, Zeilen 15 - 20, war dieses Ergebnis nicht zu erwarten, sondern vielmehr mußte der Fachmann damit rechnen, daß bei einem M/P < 1,1 kein pH-Wert des Melaminpolyphosphats über 5 erhältlich sei und daß die Phosphatderivate mit solch niedrigem M/P wegen zu geringer Hitzebeständigkeit nicht als Flammschutzmittel für Kunststoffe, wie glasfaserverstärkte Polyamide und Polyester, geeignet sind.

Aufgrund der überraschenden Hitzebeständigkeit der erfindungsgemäßen Polyphosphatderivate gibt es keine Einschränkungen mehr für die Verwendung von Melaminpolyphosphaten und entsprechenden Triazinderivaten als Flammschutzmittel in Kunststoffen, insbesondere Thermoplasten. Die Reihe von Kunststoffen, in welche die erfindungsgemäßen Polyphosphatderivate eingearbeitet werden können, finden sich auf den Seiten 6 und 7 der WO 00/02869, weswegen der Inhalt dieser Seiten zum Gegenstand der vorliegenden Anmeldung gemacht wird.

Die Polyphosphate der Erfindung können vereinfacht durch die folgende allgemeine Formel wiedergegeben werden: 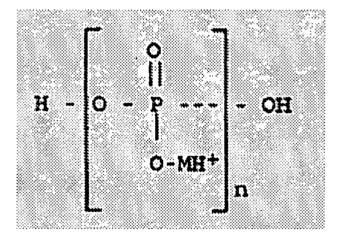 worin M eine 1,3,5-Triazinverbindung und n den mittleren Kondensationsgrad bedeutet. Für hohe und mittlere Kondensationsgrade n läßt sich die Summenformel auf (MHPO₃)ₙ reduzieren. Hierin bedeutet M wiederum die 1,3,5-Triazinverbindung und n den mittleren Kondensationsgrad.

Als 1,3,5-Triazinverbindungen kommen beispielsweise 2,4,6-Triamin-1,3,5-triazin (Melamin) sowie dessen Abkömmlinge, Kondensationsprodukte, wie Melam, Melem, Melon, Ammeline, Ammelide, aber auch 2-Ureidomelamin, Acetoguanamin, Benzoguanamin, Diaminophenyltriazin in Betracht. Selbstverständlich können auch Mischungen dieser 1,3,5-Triazinverbindungen eingesetzt werden. Melamin, seine Derivate und Kondensationsprodukte sind bei der vorliegenden Erfindung bevorzugt, insbesondere Melamin.

Den mittleren Kondensationsgrad n der Phosphate kann man nach bekannten Methoden ermitteln, wie mit Hilfe von NMR, J. Am. Chem. Soc. 78, 5715 (1956). Der mittlere Kondensationsgrad n liegt vorzugsweise mindestens bei 30, stärker bevorzugt im Bereich von 40 bis 150. Diesen mittleren Kondensationsgrad kann man auch als mittlere Kettenlänge des Phosphatderivats bezeichnen.

Die erfindungsgemäßen Polyphosphatderivate haben eine erhöhte Hitzebeständigkeit bei 320° bis 370°C. Diese wird durch die Temperatur bestimmt, bei welcher der Gewichtsverlust 2% beträgt. Das niedrige M/P-Verhältnis hat einen höheren P-Gehalt und damit eine um 15% bessere Verfahrenswirkung zur Folge und eine äußerst geringe Wasserlöslichkeit, was von größter Bedeutung für Kunststoffprodukte, insbesondere im Außenbereich, ist. Die Wasserlöslichkeit der erfindungsgemäßen Polyphosphatderivate liegt unter 0,1 g/100 ml und bevorzugt unter 0,01 g/100 ml. Bei diesen Größenordnungen kann man von einem in Wasser nahezu unlöslichen Produkt sprechen.

Das Molverhältnis M/P liegt, wie erwähnt, bevorzugt unterhalb 1,0 und stärker bevorzugt zwischen 0,8 und 1,0.

Der pH-Wert wird in einer 10 Gew.-%igen wäßrigen Aufschlämmung des erfindungsgemäßen Polyphosphatderivates bestimmt, indem 25g des Polyphosphatderivates und 225g reines Wasser von 25°C in einem Gefäß gerührt werden und mit üblichen Mitteln der pH-Wert der entstandenen wäßrigen Suspension bestimmt wird. Bevorzugte pH-Werte liegen etwa im Bereich von 5,1 bis 6,9.

Vorzugsweise haben die Polyphosphatderivate nach der Erfindung eine Zersetzungstemperatur oberhalb 360, besonders oberhalb 380, insbesondere oberhalb 400°C.

Ein erfindungsgemäßes Verfahren zur Herstellung der in den Ansprüchen 1 bis 6 beschriebenen Polyphosphatderivate besteht darin, daß man ein Orthophosphat und/oder wenigstens ein kondensiertes Phosphat einer 1,3,5-Triazinverbindung, vorzugsweise von Melamin, mit einem mittleren Kondensationsgrad n unter 20 unter einer Ammoniakatmosphäre bei einer Temperatur im Bereich von 300 bis 400°C, vorzugsweise im Bereich von 340 bis 380°C, besonders bevorzugt im Bereich von 370 bis 380°C tempert und dabei ein Kondensationsprodukt mit einem mittleren Kondensationsgrad n (Zahlenmittel) > 20 bekommt.

Gewöhnlich wird man von dem Melaminorthophosphat ausgehen, kann dieses aber ergänzen oder austauschen gegen kondensierte Phosphate, wie beispielsweise Pyrophosphate und geringer kondensierte Polyphosphate. Das Tempern muß je nach dem verwendeten Ausgangsphosphat unterschiedlich lange mit unterschiedlicher Temperatur erfolgen, so daß jedenfalls der mittlere Kondensationsgrad über 20 liegt. Die Konzentration des Ammoniaks in der Gasatmosphäre der Temperzone liegt zweckmäßig im Bereich von 0,1 bis 100 Masse-%, vorzugsweise im Bereich von 1 bis 30%, besonders im Bereich von 2 bis 10%, insbesondere von 3 bis 5%. Besonders zweckmäßig ist es, als Ausgangsmaterial eine 1,3,5-Triazinverbindung, vorzugsweise eine Melaminverbindung mit einer möglichst feinen Teilchengröße, zweckmäßig einer durchschnittlichen Teilchengröße ≤ 15 µm, vorzugsweise ≤ 10 µm, zu verwenden. Bei groberer Teilchengröße erfolgt zweckmäßig vor dem Tempern ein Mahlen der Ausgangsprodukte, um auf die genannten durchschnittlichen Teilchengrößen ≤ 15 µm, vorzugsweise ≤ 10 µm zu kommen.

Die Flammschutzeigenschaften der erfindungsgemäßen Polyphosphatderivate einer 1,3,5-Triazinverbindung können weiter gesteigert werden, wenn man die Polyphosphatderivate mit wenigstens einem Phosphinat und/oder Diphosphinat vereinigt.

Vorzugsweise besitzen die Phosphinate bzw. Diphosphinate die folgenden Formeln I bzw. II: 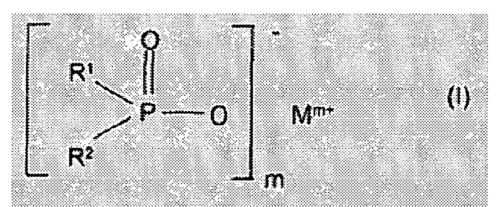 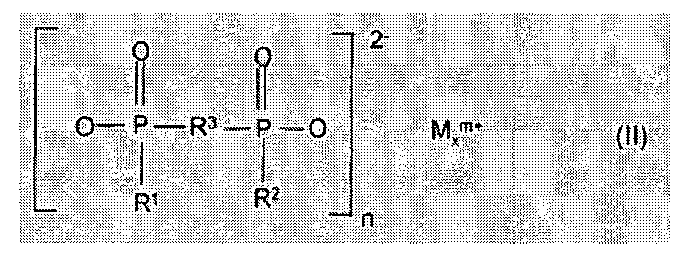 worin
- R¹, R²: gleich oder verschieden sind und C₁-C₆-Alkyl, linear oder verzweigt, C₁-C₇- Hydroxyalkyl, linear oder verzweigt oder Aryl,
- R³: C₁-C₁₀-ALkylen, linear oder verzweigt, C₆-C₁₀-Arylen, Alkylarylen oder Arylalkylen,
- M: Mg, Ca, Al, Sb, Sn, Ge, Ti, Zn, Fe, Zr, Ce, Bi, Sr, Mn, Li, Na, K und/oder eine protonierte Stickstoffbase, vorzugsweise Ca, Mg, Al und Zn,
- m: 1 bis 4,
- n: 1 bis 4,
- x: 1-4
bedeuten.

Besonders bevorzugt ist M gleich Al. Zweckmäßig enthalten die Flammschutzgemische von dem erfindungsgemäßen Polyphosphatderivat (A) und von dem Diphosphinat (B) so viel, daß A:B = 3:7 bis 7:3, vorzugsweise 3:4 bis 4:3 beträgt.

Die Flammschutzeigenschaften der erfindungsgemäßen Polyphosphatderivate einer 1,3,5-Triazinverbindung können ganz besonders bevorzugt auch dadurch gesteigert werden, daß man die Polyphosphatderivate mit Aluminium-Phosphinaten der allgemeinen Formel (I) mischt, 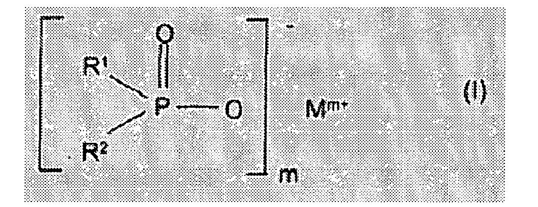 worin
- R¹: -CH₃, -CH₂OH, -C₂H₅, -CH(OH)CH₃ oder -C(OH)(CH₃)₂,
- R²: -CH₂OH, -C₂H₄OH, -C₃H₆OH oder -CH(OH)CH₃
- M: Al,
- m: 1 bis 4
bedeuten.

Die Aluminiumphosphinatgemische können dadurch erhalten werden, daß man in bekannter Weise aus Alkaliphosphinatgemischen die freien Phosphinsäuren in wässriger Lösung gewinnt und sie durch anschließendes Kochen mit Aluminiumhydroxid- oder Aluminiumoxyhydroxid-Aufschlämmungen in Wasser umsetzt. Es zeigt sich jedoch, daß diese Neutralisationsreaktion vielfach längere Reaktionszeiten beansprucht. Es ist deshalb sinnvoller, die aus der Phosphorreaktion erhaltenen Alkaliphosphinate mit der erforderlichen Menge wasserlöslicher Aluminiumsalze wie z. B. Chlorid, Hydroxychlorid, Sulfat, Nitrat oder Formiat in wäßriger Lösung zur Reaktion zu bringen, wobei es erforderlich sein kann, durch einen geringen Mineralsäurezusatz die Mischung vollständig in Lösung zu bringen und anschließend durch Anheben des pH-Wertes auf 4 bis 7 die Aluminiumphosphinat-Gemische auszufällen. Anschließendes Erhitzen der wäßrigen Suspension auf Temperaturen zwischen 105 und 150 °C in Druckgefäßen erleichtert das spätere Abtrennen des schwerlöslichen Niederschlages. Der Aluminiumphosphinatniederschlag wird anschliessend filtriert, gewaschen und getrocknet.

Eine weitere vorteilhafte Ausführungsform der Erfindung ist **dadurch gekennzeichnet, daß** alle oder ein Teil der Flammschutzmittel, d. h. das oder die Polyphosphatderivate und/oder das oder die Phosphinate und/oder Diphosphinate und/oder die weiteren enthaltenen, an sich bekannten Flammschutzmittel, mit einem siliziumhaltigen Coatingmittel überzogen und/oder anderweitig modifiziert sind. Eine solche Modifizierung ist beispielsweise aus der DE 198 30 128 A1 bekannt. Bei der Modifizierung bringt man ein organofunktionelles Silan oder eine Mischung organofunktioneller Silane oder ein oligomeres Organosiloxan oder eine Mischung oligomerer Organosiloxane oder eine lösemittelhaltige Zubereitung auf der Basis monomerer Organosilane und/oder oligomerer Organosiloxane oder eine Zubereitung auf der Basis wasserlöslicher Organopolysiloxane auf ein pulverförmiges Flammschutzmittel auf und hält das Flammschutzmittel während des Coatings in Bewegung.

Vorzugsweise werden 0,05 bis 10 Gew.-% an siliziumhaltigem Coatingmittel, bezogen auf die Menge an Flammschutzmittel, eingesetzt. Zweckmäßigerweise bringt man das Coatingmittel im Laufe von 10 Sekunden bis 2 Stunden bei einer Temperatur von 0 bis 200 °C auf das Flammschutzmittel auf. Es ist weiterhin vorteilhaft, wenn man das mit Coatingmittel umhüllte Flammschutzmittel unter Wärmeeinwirkung bei einer Temperatur bis 200 °C und/oder vermindertem Druck nachbehandelt. Als organofunktionelles Silan setzt man mit Vorteil ein aminoalkyl- oder epoxyalkyl- oder acryloxyalkyl- oder methacryloxyalkyl- oder mercaptoalkyl- oder alkenyl- oder alkylfunktionelles Alkoxysilan ein.

Ein besonderer Vorteil der Modifizierung des Flammschutzmittels mit siliziumhaltigem Coatingmittel ist, daß das Flammschutzmittel beständiger gegen Umwelteinflüsse, wie Feuchtigkeit, ist. Durch die Modifizierung wird ein Auswaschen des Flammschutzmittels im Laufe der Zeit verhindert bzw. vermindert.

Durch die folgenden Beispiele wird die Erfindung weiter erläutert.

### Beispiel 1 und Vergleichsbeispiel 1

a. 876.96 kg Melamin wurden in einem Schaufelmischer vermengt. 784 kg 75%ige Phosphorsäure wurden durch Versprühen in Berührung mit dem Melamin gebracht, wobei die Reaktion stattfand. Das gebildete Melaminorthophosphat wurde in einer ACM-Mühle fein vermahlen.
b. Das Melaminorthophosphat wurde in einem Ofen getempert. Die Ofentemperatur wurde auf 380°C eingestellt, bevor das Melaminorthophosphat in den Ofen eingeführt wurde. Die Temperatur und die Ammoniakkonzentration in der Ofen-Atmosphäre wurden jeweils eingestellt und während des gesamten Versuchs etwa konstant gehalten. In dem Ofen wurde eine Ammoniakatmosphäre geschaffen, in der die Ammoniakkonzentration veränderbar war.

Im Beispiel 1 nach der Erfindung wurde eine relativ hohe Ammoniakkonzentration, nämlich entsprechend -3 mm Wassersäule eingestellt. Ein Ergebnis war, daß der pH-Wert bei 5,524 und damit deutlich über 5 lag. Gleichzeitig war die Löslichkeit mit 0,032 g/100 ml auf etwa ein Drittel gegenüber dem nachfolgenden Vergleichsbeispiel verringert. Chemischer Angriff auf die Formwerkzeuge bei der späteren Verarbeitung konnte nicht festgestellt werden. Die Hitzebeständigkeit des Produkts des Beispiels 1 lag bei etwa 373°C-TGA (2% Gewichtsverlust), d.h. ein Gewichtsverlust von 2% wurde bei ca. 373°C erzielt.

Im Vergleichsbeispiel 1 wurde durch Veränderung der Ammoniakkonzentration in der Ofen-Atmosphäre eine niedrigere Ammoniakkonzentration als im Beispiel 1 eingestellt. Diese entsprach -100 mm Wassersäule. Der Effekt war der, daß der pH-Wert nunmehr sprunghaft nach unten ging und in der Suspension bei etwa 3,57 lag. Die Hitzebeständigkeit lag bei etwa 353°C-TGA (2% Gewichtsverlust).

Die Bedingungen und Ergebnisse dieses Experiments sind in Tabelle 1 wiedergegeben.

**Tabelle 1**

| Eigenschaften | Einheiten | Beispiel 1 | Vergleichsbeispiel 1 |
|---|---|---|---|
| P₂O₅ | % | 34,46 | 33,120 |
| Stickstoff (gesamt) | % | 41,31 | 40,120 |
| pH-Wert | | 5,524 | 3,568 |
| Löslichkeit | g/100 ml | 0,032 | 0,095 |
| Säureindex | mg KOH/g | 1,30 | 1,31 |
| Feuchtigkeit | % | 0,068 | 0,075 |
| TGA 2% Gewichtsverlust | °C | 372,91 | 353,030 |
| Zersetzungsspitze | °C | 406,11 | 397,400 |
| M/P | °C | 1,02 | 1,030 |

### Beispiel 2 und Vergleichsbeispiel 2

Melaminpolyphosphat nach der Erfindung (Beispiel 2) und außerhalb des Anspruchs 1 der Erfindung (Vergleichsbeispiel 2) wurden wie im Beispiel 1 bearbeitet. Die entsprechenden Parameter wie im Beispiel 1 wurden ermittelt und ergeben die nachfolgende Aufstellung.

Die Bedingungen und Ergebnisse dieses Experiments sind in Tabelle 2 wiedergegeben.

**Tabelle 2**

| Eigenschaften | Einheiten | Beispiel 2 | Vergleichsbeispiel 2 |
|---|---|---|---|
| P₂O₅ | % | 35,820 | 30,680 |
| Stickstoff (gesamt | % | 41,190 | 44,690 |
| pH-Wert | | 5,470 | 3,580 |
| Löslichkeit | g/100 ml | 0,002 | 0,017 |
| Säureindex | mg KOH/g | 0,435 | 3,271 |
| Feuchtigkeit | % | | 0,313 |
| TGA 2% Gewichtsverlust | °C | 373,100 | 374,770 |
| Zersetzungsspitze | °C | 407,430 | 406,260 |
| M/P | °C | 0,980 | 1,240 |

In diesem Fall war die Hitzebeständigkeit in beiden Versuchen praktisch gleich, der pH-Wert beim Beispiel 2 nach der Erfindung aber erheblich höher als bei Vergleichsbeispiel 2.

## Patentansprüche

1. Polyphosphatderivat einer 1,3,5-Triazinverbindung, vorzugsweise Melaminpolyphosphat, mit
a) einem mittleren Kondensationsgrad n (Zahlenmittel) > 20
b) mit einem pH-Wert einer 10%igen Aufschlämmung des Polyphosphatderivates in Wasser bei 25°C von 5 oder höher,
c) einem Molverhältnis von 1,3,5-Triazinverbindung zu Phosphor (M/P) < 1,1,
d) einer Zersetzungstemperatur > 320°C, bei welcher der Gewichtsverlust 2% beträgt, und
e) einer Löslichkeit < 0,1 g/100 ml.

2. Polyphosphatderivat nach Anspruch 1, **dadurch gekennzeichnet, daß** es
e) eine Löslichkeit < 0,01 g/100 ml hat.

3. Polyphosphatderivat nach Anspruch 1 oder 2, **dadurch gekennzeichnet, daß** es ein Molverhältnis (c) von 1,3,5-Triazinverbindung zu Phosphor < 1,0, vorzugsweise im Bereich zwischen 0,8 und 1,0 hat.

4. Polyphosphatderivat nach einem der Ansprüche 1 bis 3, **dadurch gekennzeichnet, daß** eine 10%ige Aufschlämmung des Polyphosphatderivates in Wasser bei 25°C (b) einen pH-Wert im Bereich von 5,1 bis 6,9 hat.

5. Polyphosphatderivat nach einem der Ansprüche 1 bis 4, **dadurch gekennzeichnet, daß**
a) sein mittlerer Kondensationsgrad (Zahlenmittel) > 30, besonders bei 40 bis 150 liegt.

6. Polyphosphatderivat nach einem der Ansprüche 1 bis 5, **dadurch gekennzeichnet, daß**
d) seine Zersetzungstemperatur höher als 360, vorzugsweise höher als 380, besonders höher als 400°C ist.

7. Verfahren zur Herstellung eines Polyphosphatderivates einer 1,3,5-Triazinverbindung nach einem der Ansprüche 1 bis 6, **dadurch gekennzeichnet, daß** man ein Orthophosphat oder ein kondensiertes Phosphat einer 1,3,5-Triazinverbindung mit einem mittleren Kondensationsgrad n (Zahlenmittel) unter 20 in einer Ammoniakatmosphäre bei einer Temperatur im Bereich von 300 bis 400°C tempert, bis der mittlere Kondensationsgrad über 20 und das Molverhältnis von 1,3,5-Triazinverbindung zu Phosphor (M/P) unter 1,1 liegt.

8. Verfahren nach Anspruch 7, **dadurch gekennzeichnet, daß** man in einem Temperaturbereich von 340 bis 380, vorzugsweise von 370 bis 380°C tempert.

9. Verfahren nach Anspruch 7 oder 8, **dadurch gekennzeichnet, daß** man vor dem Tempern das Polyphosphatderivat auf eine durchschnittliche Teilchengröße ≤ 15 µm, vorzugsweise ≤ 10 µm vermahlt.

10. Verfahren nach einem der Ansprüche 7 bis 9, **dadurch gekennzeichnet, daß** man unter einer Ammoniakatmosphäre tempert, die Ammoniak in einer Konzentration von 0,1 bis 100, bevorzugt von 1 bis 30, besonders von 2 bis 10, insbesondere von 3 bis 5 Masse-% enthält.

11. Verwendung eines Polyphosphatderivates einer 1,3,5-Triazinverbindung nach einem der Ansprüche 1 bis 6 als Flammschutzmittel für Kunststoffe, vorzugsweise Thermoplaste und Duroplaste, insbesondere glasfaserverstärkte Polyamide, Polyester oder Polyolefine.

12. Flammschutzmittel, enthaltend wenigstens ein Polyphosphatderivat einer 1,3,5-Triazinverbindung nach einem der Ansprüche 1 bis 6.

13. Flammschutzmittel nach Anspruch 12, **dadurch gekennzeichnet, daß** es noch andere an sich bekannte Flammschutzmittel enthält.

14. Flammschutzmittel nach einem der Ansprüche 12 und 13, **dadurch gekennzeichnet, daß** es zusätzlich wenigstens ein Phosphinat und/oder Diphosphinat enthält.

15. Flammschutzmittel nach einem der Ansprüche 12 bis 14, **dadurch gekennzeichnet, daß** es Phosphinat der allgemeinen Formel I und/oder Diphosphinat der allgemeinen Formel II enthält: 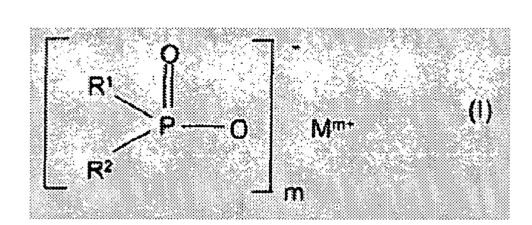 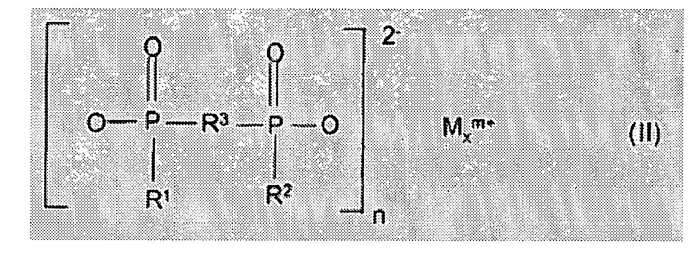 worin
R¹, R² gleich oder verschieden sind und C₁-C₆-Alkyl, linear oder verzweigt, C₁-C₇- Hydroxyalkyl, linear oder verzweigt oder Aryl,
R³ C₁-C₁₀Alkylen, linear oder verzweigt, C₆-C₁₀-Arylen, -Alkylarylen oder Arylalkylen,
M Mg, Ca, Al, Sb, Sn, Ge, Ti, Zn, Fe, Zr, Ce, Bi, Sr, Mn, Li, Na, K und/oder eine protonierte Stickstoffbase, vorzugsweise Ca, Mg, Al und Zn,
m 1 bis 4,
n 1 bis 4,
x 1-4
bedeuten.

16. Flammschutzmittel nach Anspruch 15, **dadurch gekennzeichnet, daß** M = Al ist.

17. Flammschutzmittel nach Anspruche 15, **dadurch gekennzeichnet, daß** das Phosphinat ein Phosphinat der allgemeinen Formel I ist: 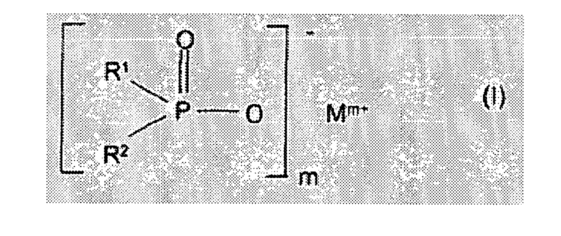 worin
R¹ -CH₃, -CH₂OH, -C₂H₅, -CH(OH)CH₃ oder -C(OH)(CH₃)₂,
R² -CH₂OH, -C₂H₄OH, -C₃H₆OH oder -CH(OH)CH₃,
M Al,
m 1 bis 4
bedeuten.

18. Flammschutzmittel nach einem der Ansprüche 12 bis 17, **dadurch gekennzeichnet, daß** das oder die Polyphosphatderivate und/oder das oder die Phosphinate und/oder Diphosphinate und/oder die weiteren enthaltenen, an sich bekannten Flammschutzmittel mit einem siliziumhaltigen Coatingmittel überzogen und/oder anderweitig modifiziert sind.

## Claims

1. A polyphosphate derivative of a 1,3,5-triazine compound, preferably melamine polyphosphate, with
a) an average condensation coefficient n (number average) > 20,
b) with a pH-value of a 10% slurry of the polyphosphate derivative in water at 25°C of 5 or higher,
c) a molar ratio of 1,3,5-triazine compound to phosphorus (M/P) < 1.1,
d) a decomposition temperature > 320°C, at which the loss of weight amounted to 2 %, and
e) a solubility < 0.1 g/100 ml

2. A polyphosphate derivative according to claim 1, **characterised in that**
e) it has a solubility < 0.1 g/100 ml.

3. A polyphosphate derivative according to claim 1 or 2, **characterised in that** it has a molar ratio (c) of 1,3,5-triazine compound to phosphorus < 1.0, preferably in the range of between 0.8 und 1.0.

4. A polyphosphate derivative according to one of claims 1 to 3, **characterised in that** a 10% slurry of the polyphosphate derivative in water at 25°C (b) has a pH-value in the range of 5.1 to 6.9.

5. A polyphosphate derivative according to one of claims 1 to 4, **characterised in that**
a) its average condensation coefficient (number average) > 30, particularly from 40 to 150.

6. A polyphosphate derivative according to one of claims 1 to 5, **characterised in that**
d) its decomposition temperature is higher than 360°C, preferably higher than 380°C, particularly higher than 400°C.

7. A process for the production of a polyphosphate derivative of a 1,3,5-triazine compound according to one of claims 1 to 6, **characterised in that** an orthophosphate or a condensed phosphate of a 1,3,5-triazine compound with an average condensation coefficient n (number average) below 20 is heat-treated in an ammonia atmosphere at a temperature in the range of 300 to 400°C until the average condensation coefficient is above 20 and the molar ratio of 1,3,5-triazine compound to phosphorus (M/P) is below 1.1.

8. A process according to claim 7 **characterised in that** the heat treatment is effected in a temperature range of 340 to 380, preferably 370 to 380°C.

9. A process according to claim 7 or 8 **characterised in that** prior to the heat treatment the polyphosphate derivative is ground to an average particle size ≤ 15 µm, preferably ≤ 10 µm.

10. A process according to one of claims 7 to 9 **characterised in that** the heat treatment is effected in an ammonia atmosphere which contains ammonia in a concentration of 0.1 to 100, preferably 1 to 30, particularly 2 to 10, in particular 3 to 5 % by mass.

11. Use of a polyphosphate derivative of a 1,3,5-triazine compound according to one of claims 1 to 6 as a flame-retardant agent for plastic materials, preferably thermoplastic materials and thermosetting materials, in particular glass fibre-reinforced polyamides, polyesters or polyolefins.

12. A flame-retardant agent including at least one polyphosphate derivative of a 1,3,5-triazine compound according to one of claims 1 to 6.

13. A flame-retardant agent according to claim 12 **characterised in that** it also contains other per se known flame-retardant agents.

14. A flame-retardant agent according to one of claims 12 and 13 **characterised in that** it additionally contains at least one phosphinate and/or diphosphinate.

15. A flame-retardant agent according to one of claims 12 to 14 **characterised in that** it contains phosphinate of the general formula I and/or diphosphinate of the general formula II: 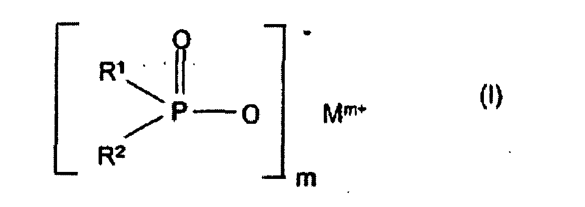 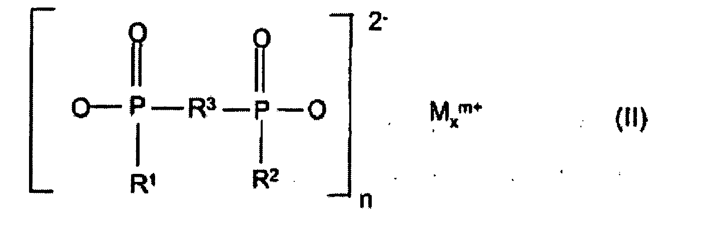 wherein
R¹, R² are the same or different and C₁-C₆-alkyl, straight or branched, C₁-C₇- hydroxyalkyl, straight or branched, or aryl,
R³ is C₁-C₁₀-alkyls, straight or branched, C₆-C₁₀-aryls, alkylaryls or arylalkyls,
M is Mg, Ca, Al, Sb, Sn, Ge, Ti, Zn, Fe, Zr, Ce, Bi, Sr, Mn, Li, Na, K and/or a protonised nitrogen base, preferably Ca, Mg, Al and Zn,
m is 1 to 4,
n is 1 to 4, and
x is 1 - 4.

16. A flame-retardant agent according to claim 15 **characterised in that** M = Al.

17. A flame-retardant agent according to claim 15 **characterised in that** the phosphinate is a phosphinate of the general formula I: 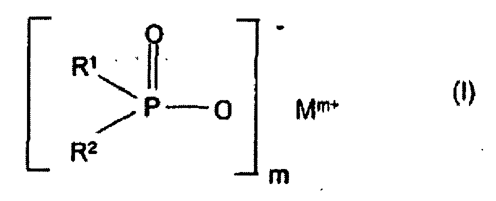 wherein
R¹ is -CH₃, -CH₂OH, -C₂H₅, -CH(OH)CH₃ or -C(OH)(CH₃)₂,
R² is -CH₂OH, -C₂H₄OH, -C₃H₆OH or -CH(OH)CH₃,
M is Al, and
m 1 to 4

18. A flame-retardant agent according to one of claims 12 to 17 **characterised in that** the polyphosphate derivative or derivatives and/or the phosphinate or phosphinates and/or diphosphinate or diphosphinates and/or the further included per se known flame-retardant agents are coated with a silicon-bearing coating agent and/or modified in some other fashion.

## Revendications

1. Dérivé de polyphosphate d'un composé de triazine 1, 3, 5, de préférence polyphosphate de mélamine, avec
a) un degré de condensation moyen n (moyenne chiffrée) > 20,
b) avec une valeur pH d'une suspension à 10% du dérivé de polyphosphate dans l'eau à 25 °C de 5 ou plus,
c) une proportion molaire de composé de triazine 1, 3, 5 par rapport au phosphore (M/P) < 1,1,
d) une température de décomposition > 320 °C, à laquelle la perte de poids est de 2%
e) une solubilité < 0,1 g/100 ml.

2. Dérivé de polyphosphate selon la revendication 1, **caractérisé en ce qu'**il a
a) une solubilité < 0,01 g/100 ml.

3. Dérivé de polyphosphate selon la revendication 1 ou 2, **caractérisé en ce qu'**il a une proportion molaire (c) de composé de triazine 1, 3, 5 par rapport au phosphore < 1,0, de préférence située entre 0,8 et 1,0.

4. Dérivé de polyphosphate selon l'une des revendications 1 à 3, **caractérisé en ce qu'**une suspension à 10% du dérivé de polyphosphate dans l'eau à 25 °C (b) a une valeur pH située entre 5,1 et 6,9.

5. Dérivé de polyphosphate selon l'une des revendications 1 à 4, **caractérisé en ce que**
a) son degré de condensation moyen (moyenne chiffrée) est > 30, en particulier situé entre 40 et 150.

6. Dérivé de polyphosphate selon l'une des revendications 1 à 5, **caractérisé en ce que**
a) sa température de décomposition est supérieure à 360, de préférence supérieure à 380, en particulier de préférence supérieure à 400 °C.

7. Procédé pour fabriquer un dérivé de polyphosphate d'un composé de triazine 1, 3, 5 selon l'une des revendications 1 à 6, **caractérisé en ce qu'**on étuve un orthophosphate ou un phosphate condensé d'un composé de triazine 1, 3, 5 avec un degré de condensation moyen n (moyenne chiffrée) inférieur à 20 dans une atmosphère d'ammoniaque à une température située entre 300 et 400 °C jusqu'à ce que le degré de condensation moyen soit supérieur à 20 et la proportion molaire de composé de triazine 1, 3, 5 par rapport au phosphore (M/P) inférieure à 1,1.

8. Procédé selon la revendication 7, **caractérisé en ce qu'**on étuve à une température située entre 340 et 380, de préférence entre 370 et 380 °C.

9. Procédé selon la revendication 7 ou 8, **caractérisé en ce qu'**on moud le dérivé de polyphosphate à une taille de grain moyenne ≤ 15 µm, de préférence ≤ 10 µm, avant de l'étuver.

10. Procédé selon l'une des revendications 7 à 9, **caractérisé en ce qu'**on étuve sous une atmosphère d'ammoniaque, dont la concentration en ammoniaque est située entre 0,1 et 100, de préférence entre 1 et 30, en particulier de préférence entre 2 et 10, plus particulièrement de préférence entre 3 et 5% de masse.

11. Utilisation d'un dérivé de polyphosphate d'un composé de triazine 1, 3, 5 selon l'une des revendications 1 à 6 comme moyen ignifuge pour des plastiques, de préférence des thermoplastiques et duroplastiques, en particulier des polyamides renforcés par de la fibre de verre, des polyesters ou des polyoléfines.

12. Moyen ignifuge contenant au moins un dérivé de polyphosphate d'un composé de triazine 1, 3, 5 selon l'une des revendications 1 à 6.

13. Moyen ignifuge selon la revendication 12, **caractérisé en ce qu'**il contient encore d'autres moyens ignifuges connus en soi.

14. Moyen ignifuge selon l'une des revendications 12 et 13, **caractérisé en ce qu'**il contient en plus au moins un phosphinate et/ou un diphosphinate.

15. Moyen ignifuge selon l'une des revendications 12 à 14, **caractérisé en ce qu'**il contient du phosphinate de la formule générale 1 et/ou du disphosphinate de la formule générale II. 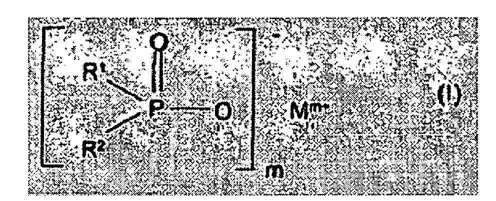 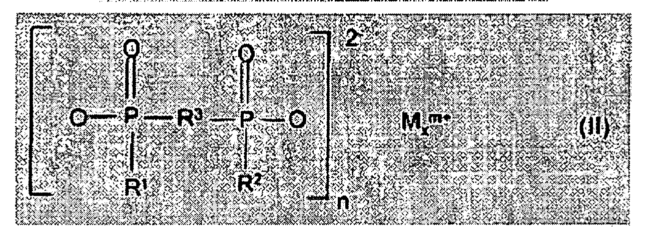 où
R¹, R² semblables ou différents et C₁-C₆-alkyle, linéaire ou ramifié, C₁-C₇-hydroxyalkyle, linéaire ou ramifié ou aryle,
R³ C₁-C₁₀-alkylène, linéaire ou ramifié, C₆-C₁₀-arylène, -alkylarylène ou arylalkylène
M Mg, Ca, Al, Sb, Sn, Ge, Ti, Zn, Fe, Zr, Ce, Bi, Sr, Mn, Li, Na, K et/ou une base azotée protonisée, de préférence Ca, Mg, Al et Zn.
m 1 à 4
n 1 à 4
x 1 - 4

16. Moyen ignifuge selon la revendication 15, **caractérisé en ce que** M = Al.

17. Moyen ignifuge selon la revendication 15, **caractérisé en ce que** le phosphinate est un phosphinate de la formule générale I : 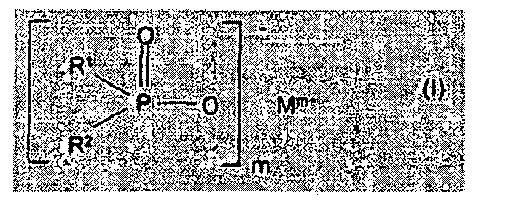 où
R¹ -CH₃, -CH₂OH -C₂H₅, -CH(OH)CH₃ ou -C(OH)(CH₃)₂,
R² -CH₂OH -C₂H₄OH, -C₃H₆OH ou -CH(OH)CH₃,
M Al,
M 1 à4.

18. Moyen ignifuge selon l'une des revendications 12 à 17, **caractérisé en ce que** le ou les dérivés de polyphosphate et/ou le ou les phosphinates et/ou diphosphinates et/ou les autres moyens ignifuges connus en soi sont recouverts d'un moyen de revêtement contenant du silicium et/ou autrement modifiés.
